**Europäisches Patentamt**

**European Patent Office**

**Office européen des brevets**

(19)

(11) Veröffentlichungsnummer: **0 413 043 A1**

# (12) EUROPÄISCHE PATENTANMELDUNG

(21) Anmeldenummer: **89115112.8**

(22) Anmeldetag: **16.08.89**

(51) Int. Cl.5: **A61B 6/14**

(43) Veröffentlichungstag der Anmeldung:
**20.02.91 Patentblatt 91/08**

(84) Benannte Vertragsstaaten:
**DE FR GB IT SE**

(71) Anmelder: **Siemens Aktiengesellschaft**
**Wittelsbacherplatz 2**
**D-8000 München 2(DE)**

(72) Erfinder: **Pfeiffer, Joachim, Dr.**
**Eifelstrasse 33**
**D-6140 Bensheim(DE)**
Erfinder: **Schulze-Ganzlin, Dipl.-Ing. (TH)**
**Seehofstrasse 1**
**D-6143 Lorsch(DE)**
Erfinder: **Schwotzer, Axel, Dipl.-Ing. (TH)**
**Georgenstrasse 32**
**D-6087 Büttelborn(DE)**

(54) Zahnärztliche Röntgendiagnostikeinrichtung.

(57) Es wird eine zahnärztliche Röntgendiagnostik-einrichtung vorgeschlagen, mit der es möglich ist, unter Vermeidung von Verstärkerfolien Aufnahmen mit verminderter Streustrahlung und höherer Bild-schärfe machen zu können. Zur Konvertierung der Röntgen-Strahlen ist eine gitterartig strukturierte Szintillatorplatte (10) vorgesehen, die eine Vielzahl flächiger und sich in Strahlungsrichtung erstreckender Szintillatorelemente (17) enthält, welche durch lichtdichte Wandungen (18) voneinander getrennt sind. Alternativ kann eine unstrukturierte Szintillator-platte (25) aus transparentem Szintillatormaterial vor-gesehen sein, an die sich eine gitterartig strukturierte Faseroptik (26) mit relativ kleiner numerischer Aper-tur anschließt, die aus einer Vielzahl von flächigen und sich räumlich in Strahlungsrichtung erstrecken-den Einzelelementen (27) bestehen kann.

FIG 3

EP 0 413 043 A1

# ZAHNÄRZTLICHE RÖNTGENDIAGNOSTIKEINRICHTUNG

Bei zahnärztlichen Röntgendiagnostikeinrichtungen, insbesondere bei sogenannten Panorama-Röntgendiagnostikeinrichtungen, ist es üblich, den lichtempfindlichen Film sandwichartig zwischen Verstärkerfolien anzuordnen, um so Aufnahmen in kürzerer Zeit und mit weniger Strahlendosis machen zu können.

Die Verstärkerfolien sind mit einer Szintillationsschicht versehen, welche auf sie auftreffende Röntgenstrahlung in sichtbare Strahlung umsetzt.

Abgesehen davon, daß die Verstärkerfolien relativ teuer sind, läßt sich mit der vorbeschriebenen Film-Folien-Technik keine hinreichend gute Bildschärfe erzielen, insbesondere, weil die Strahlen innerhalb der Folie nach allen Seiten divergieren, wodurch innerhalb der Schicht unerwünschte Reflexionen auftreten, die letztlich zu einer Verschlechterung der Ortsauflösung und damit zu einer Verschlechterung der Bildschärfe führen.

In der US-PS 4,305,989 ist eine Röntgendiagnostikeinrichtung beschrieben, bei der zur Erzielung einer höheren Bildauflösung und Vermeidung von Verstärkerfolien vorgeschlagen wird, im Anschluß an den Sekundärschlitz ein ortfestes Bildverstärkersystem anzuordnen, an dem der lichtempfindliche Film (ohne Verstärkerfolie) vorbeigezogen wird.

Das Bildverstärkersystem enthält eine unmittelbar an den Sekundärschlitz angrenzende Platte mit einer Vielzahl von mikrofeinen, hohlen Glaszylindern, die in Strahlungsrichtung ausgerichtet sind und deren Zwischenräume mit Bleiglas ausgefüllt sind. Ein- und Ausgänge dieser Platte sind mit einer elektrisch lei tenden Schicht überzogen, wodurch die Glaszylinder elektrisch parallel miteinander verbunden sind. In der Platte wird die Röntgenstrahlung in Elektronen umgewandelt. Durch ein zwischen den Schichten angelegtes Spannungspotential wird in jedem der Mikrokanäle ein elektrostatisches Feld erzeugt, welches bewirkt, daß jedes erzeugte Elektron vor Austritt aus der Platte vervielfacht wird (kaskadenartiger Vorgang). Die aus der Platte austretenden Elektronen werden auf eine sich daran anschließende Leuchtschicht geworfen, wo sie in sichtbare Strahlung umgewandelt werden. Die sichtbare Strahlung wird schließlich über eine Faseroptik auf den lichtempfindlichen Film geleitet, der mit Hilfe von Antriebs- und Andruckrollen an der Faseroptik anliegend vorbeibewegt wird.

Alternativ zu der erläuterten Anordnung ist in der Druckschrift angegeben, daß vor Eintritt der Röntgenstrahlung in die mikrofeinen Glaszylinder eine Konversionsschicht aus geeignetem Material vorgesehen sein kann, welche die Röntgenstrahlung vor Eintritt in die mikrofeinen Glaszylinder in Elektronen umwandelt, oder welche die Röntgenstrahlung zunächst in sichtbare Strahlung und diese dann wieder in Elektronen umwandelt.

Das bei dem vorgenannten Stand der Technik vorgesehene Bildverstärkersystem ist im Hinblick auf die Anzahl der vorgesehenen Komponenten und verwendeten unterschiedlichen Materialien in der Herstellung relativ aufwendig.

Ziel der vorliegenden Erfindung ist es, demgegenüber eine Verbesserung, insbesondere Vereinfachung zu erzielen, also eine zahnärztliche Röntgendiagnostikeinrichtung anzugeben, mit welcher sich mit geringerem Aufwand Aufnahmen mit verminderter Streustrahlung und höherer Bildschärfe erstellen lassen.

Das angestrebte Ziel kann gemäß der Erfindung auf zwei Wegen erreicht werden, zum einen, indem zur Konversion der Röntgenstrahlung eine gitterartig strukturierte Szintillatorplatte vorgesehen ist, die eine Vielzahl flächiger und sich in Strahlungsrichtung erstreckender Szintillatorelemente enthält, welche durch lichtdichte Wandungen voneinander getrennt sind, und zum anderen, indem eine unstrukturierte Szintillatorplatte aus transparentem Szintillatormaterial vorgesehen ist, an der sich eine gitterartig strukturierte Faseroptik, die aus einer Vielzahl von flächigen und sich räumlich in Strahlungsrichtung erstreckenden Einzelelementen besteht, anschließt.

In beiden Fällen erfolgt eine direkte Konversion der Röntgenstrahlen in sichtbare Strahlung, an der sich die Bilderzeugung in Form eines lichtempfindlichen Filmes oder eines Bilddetektors anschließen kann. Im Falle eines lichtempfindlichen Filmes kann dieser, wie nachstehend erläutert, relativ zur Strahlenquelle bewegt werden, so daß eine sogenannte Panorama-Röntgenaufnahme erstellt werden kann; ebenso kann auch der Film stationär bleiben, wie dies für Einzelaufnahmen üblich ist.

Vorteilhafte Ausgestaltungen und Weiterbildungen der Erfindung sind in den Unteransprüchen enthalten und ergeben sich aus der nachstehenden Beschreibung eines Ausführungsbeispieles. Es zeigen:

FIG 1 eine Prinzipdarstellung einer zahnärztlichen Röntgendiagnostikeinrichtung zur Erstellung einer Panorama-Röntgenaufnahme in perspektivischer Ansicht,

FIG 2 einen Teil der Einrichtung nach FIG 1 im Horizontalschnitt,

FIG 3 Aufbau der Szintillatorplatte in perspektivischer Darstellung,

FIG 4 die Szintillatorplatte nach FIG 3 im ungefüllten Zustand,

FIG 5 bis 7 verschiedene Varianten einer möglichen Gitterstruktur für die Szintillatorplatte und FIG 8 und 9 eine Alternativ-Version zu der in FIG 3/4 gezeigten Ausführung zur Konversion der Röntgenstrahlung.

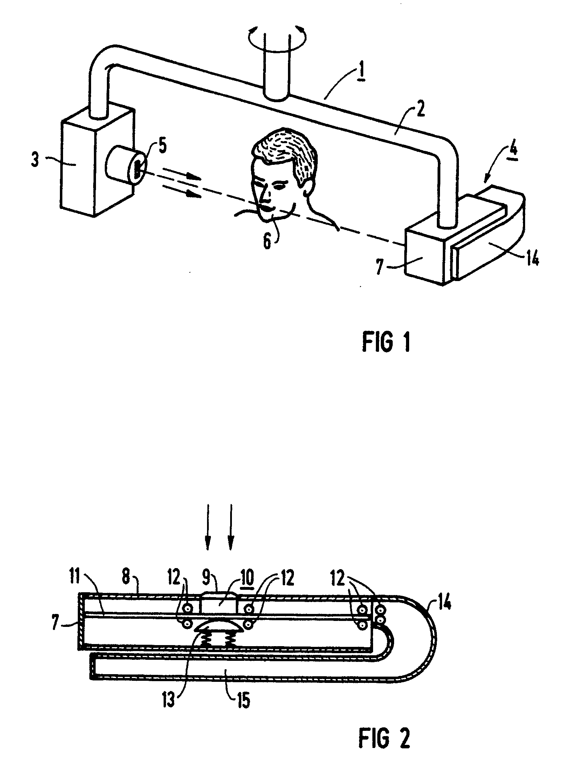

Die FIG 1 zeigt in stark vereinfachter perspektivischer Darstellung die wesentlichen Teile einer zahnärztlichen Röntgendiagnostikeinrichtung zur Erstellung einer sogenannten Panorama-Schichtaufnahme. Die Einrichtung enthält in bekannter Weise eine um eine lotrechte Achse schwenkbare Dreheinheit 1, die einen Tragarm 2 enthält, an dessen einem Ende ein Röntgenstrahler 3 und an dessen anderem Ende eine Strahlenaufnahmeeinheit 4 gehalten sind. Das am Strahlenaustrittsfenster 5 des Röntgenstrahlers 3 austretende Strahlenbündel wird nach Durchdringen des Aufnahmeobjekts (Patientenkopf 6) von der Strahlenaufnahmeeinheit 4 erfaßt und dort entsprechend dem vorhandenen Strahlenbild in ein sichtbares Bild umgewandelt. Die Dreheinheit 1 ist in bekannter Weise um den Patientenkopf schwenkbar, wodurch sich eine Übersichtaufnahme von dem Objekt, z.B. vom Kiefer eines Patienten, erstellen läßt.

Die Strahlenaufnahmeeinheit 4, die in FIG 2 stark vereinfacht in einem Horizontalschnitt dargestellt ist, enthält ein erstes, mit dem Tragarm 2 fest verbundenes Gehäuse 7, welches in bekannter Weise eine Sekundärblende 8 mit einer dem Strahlenaustrittsfenster 5 korrespondierend angeordneten Schlitzöffnung 9 enthält. Unmittelbar im Anschluß an die Sekundärblende 8 ist eine Szintillatorplatte 10 befestigt, deren Aufbau später noch näher erläutert wird. Ein lichtempfindlicher Film 11 wird mittels diverser Antriebsrollen 12 und einer federnd anliegenden Andruckplatte 13 an der Szintillatorplatte 10 vorbeibewegt. Der Film wird nach Belichtung in ein zweites Gehäuse 14 transportiert, welches am ersten Gehäuse 7 lichtdicht abnehmbar gehalten ist. Auch in diesem Gehäuse sind diverse Antriebsrollen vorhanden, die den Film nach der Belichtung in den schmalen rückwärtigen Schacht 15 des Gehäuses 14 transportieren.

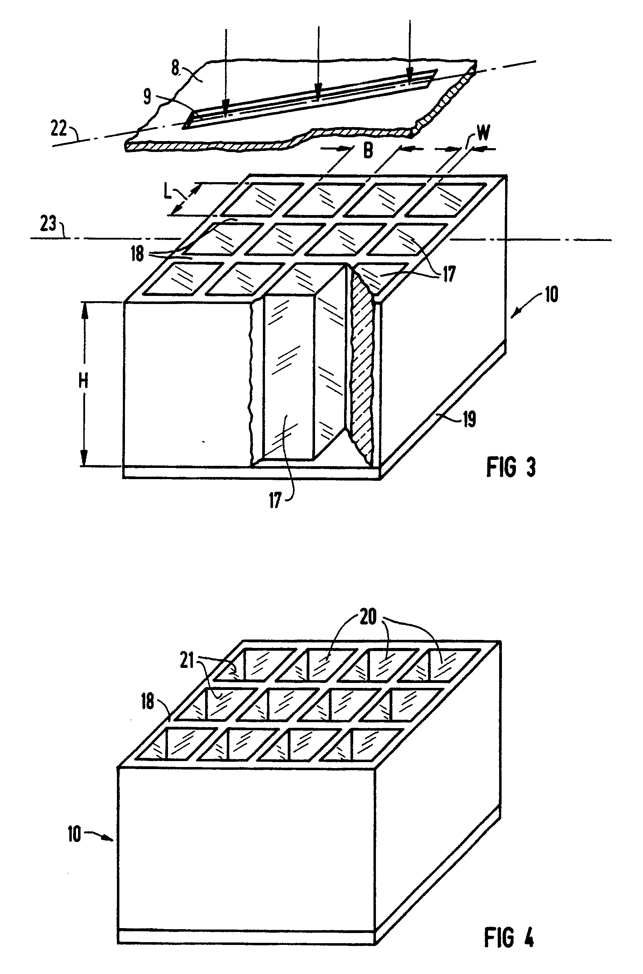

Die FIG 3 zeigt in einer perspektivischen Darstellung den Auf bau der Szintillatorplatte 10 sowie deren Zuordnung zu dem schematisch darübergezeichneten Schlitz 9 der Sekundärblende 8.

Die Szintillatorplatte 10 besteht aus einer Vielzahl von einzelnen Szintillatorelementen 17 mit quadratischem oder rechteckigem Querschnitt, welche durch lichtdichte Wandungen 18 voneinander getrennt sind und so eine gitterartige Struktur bilden. Die Szintillatorelemente 17 weisen vorteilhafterweise eine Kantenlänge (L/B) von z.B. 50 $\mu$m auf, die Breite (W) der dazwischenliegenden Wandungen beträgt 5 $\mu$m. Die Höhe (H) der gesamten Szintillatorplatte beträgt z.B. 300 $\mu$m. Aus den genannten Abmessungen ist zu entnehmen, daß die Strahleneintrittsfläche sämtlicher Szintillatorelemente im Verhältnis zur Fläche der sie begrenzenden Wandungen sehr groß ist. Vorzugsweise beträgt das Größenverhältnis 10 : 1. Die Höhe H der Szintillatorplatte beträgt mindestens das Zweifache, vorzugsweise das Zehnfache der Kantenlänge eines Szintillatorelementes.

An der dem Strahleneintritt gegenüberliegenden Seite kann der lichtempfindliche Film direkt vorbeigezogen werden, wie dies in FIG 2 dargestellt ist. Wird anstelle des lichtempfindlichen Filmes eine Detektoranordnung vorgesehen, so kann diese, wie in FIG 3 mit 19 angedeutet, sich unmittelbar an die Szintillatorplatte 10 anschließen. Eine solche Detektoranordnung kann aus ein oder mehreren Halbleiterdetektoren, z.B. CCD-Elementen, gebildet werden.

Um die Struktur vor Abrieb durch den vorbeilaufenden Film zu schützen, kann die Kontaktfläche mit einer dünnen, harten Schicht überzogen sein. Eine solche kann z.B. durch Bedampfen mit $MgF_2$ in einer Schichtdicke von etwa 5$\mu$m - 20$\mu$m geschaffen werden.

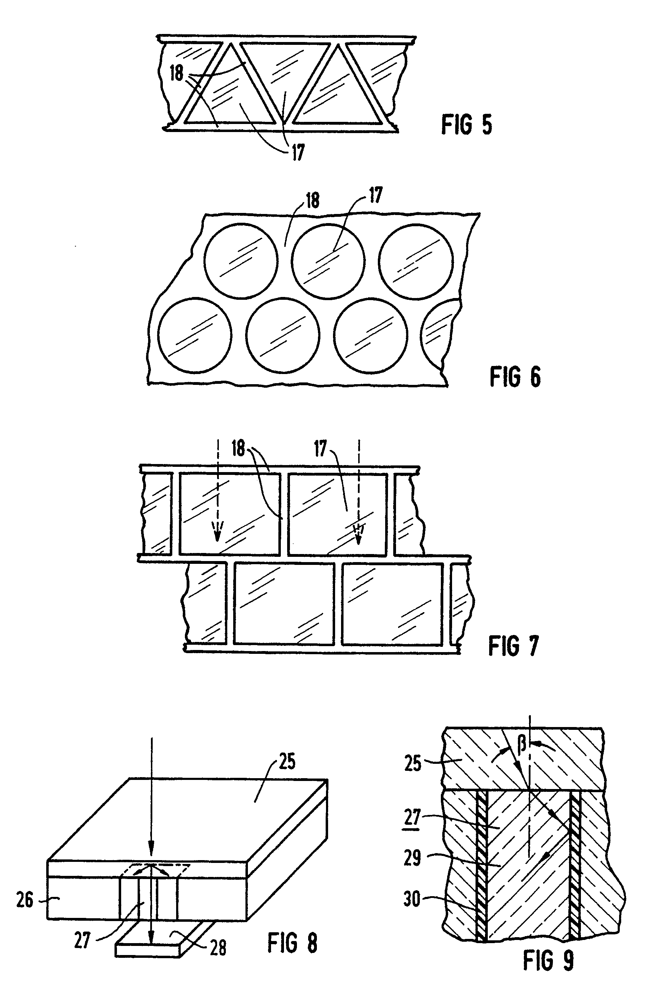

Die Szintillatorplatte 10 wird gebildet, indem in eine Platte aus lichtdichtem Material, die in ihren Abmessungen etwa den der Schlitzöffnung 9 der Sekundärblende 8 entspricht, nach einem besonderen Verfahren zur Herstellung von Mikrostrukturen längs der Strahlungsrichtung (Pfeile) verlaufende Kanäle 20 eingearbeitet werden, die so eine gitter- oder wabenförmige Struktur bilden (FIG 4). Die Kanäle 20 werden sodann mit Szintillatormaterial gefüllt und bilden so, wie aus FIG 3 ersichtlich, stabförmige Elemente. Vorteilhafterweise werden, bevor das Szintillatormaterial in die Kanäle 20 eingegeben wird, die Wandungen 21 der Kanäle mit einer lichtreflektierenden Oberfläche versehen. Zweckmäßigerweise bietet sich an, die Wandungen der Kanäle innen zu verspiegeln.

Wenn, wie in der Ausführung gemäß FIG 3 und 4, die gitterartige Struktur symmetrisch aufgebaut ist und die Trennwandungen 18 parallel zueinander verlaufen, ist es vorteilhaft, die Szintillatorplatte 10 gegenüber der Schlitzöffnung 9 um einige Winkelgrade gedreht anzuordnen. Längssymmetrieachse 22 des Sekundärschlitzes 9 bildet sodann in bezug auf die Symmetrieachse der Szintillatorplatte 10 einen Winkel von ein bis zwei Grad. In FIG 3 ist diese Beziehung stark überzeichnet dargestellt.

Der vorgenannte Versatz von Szintillatorplatte und Sekundärschlitz läßt sich vermeiden, wenn die gitter- oder wabenförmige Anordnung der Szintillatorelemente so getroffen ist, daß in oder parallel zur Laufrichtung des Filmes die nicht zur Abbildung beitragenden Wandungen der Gitterstruktur unterbrochen werden. Dies kann entweder durch eine andere Querschnittsform erreicht werden, indem der Querschnitt der Szintillatorelemente dreieck- oder kreisförmig ist, wie in FIG 5 und 6 dargestellt, oder, indem die im Querschnitt rechteckig oder quadratischen Szintillatorelemente gegeneinander (treppenartig) so versetzt sind, daß sich in der in FIG 7 durch gestrichelte Pfeile angegebenen Laufrichtung des Filmes die vorgenannten Unterbrechungen ergeben.

Alternativ zu der in den FIG 2 und 3 dargestellten Ausführungsform zur Konversion der Röntgenstrahlung in sichtbare Strahlung kann auch eine Ausführung vorgesehen werden, wie in FIG 8 im Prinzip dargestellt. Die Röntgenstrahlung wird zunächst auf eine transparente unstrukturierte Szintillatorplatte 25 geworfen, an der sich eine Faseroptik 26, die eine Struktur aufweist, wie in den FIG 3 bis 7 dargestellt, anschließt. Die Faseroptik 26 kann aus einem Bündel von einzelnen, im Querschnitt runden Fasern 27 bestehen, die, wie vorbeschrieben, gegeneinander optisch isoliert sind.

Eine Faseroptik wird üblicherweise durch eine numerische Apertur (NA) und einen Öffnungswinkel ($\beta$) charakterisiert. Der Öffnungswinkel ist der Winkel der einfallenden Strahlen zur Senkrechten auf die Faseroptik (FIG 9). Strahlen mit einem Einfallswinkel kleiner als $\beta$ treten durch die Faseroptik durch. Der Öffnungswinkel hängt neben den Brechungsindizes $n_1$ des Faserkerns (29) und $n_2$ des Fasermantels (30) auch vom Brechungsindex $n_0$ des die Faseroptik umgebenden Mediums, in diesem Fall dem Szintillator (25), ab. Der Zusammenhang ist folgender:

$$NA = n_0 * \sin\beta = (n_1^2 - n_2^2)^{0,5} .$$

Übliche Werte sind z.B.: $n_0$ = 1,8 für den Szintillator;

$n_1$ = 1,6 und

$n_2$ = 1,5 für die Faseroptik.

Der Öffnungswinkel sollte in Abhängigkeit von der Dicke des Szintillators und der angestrebten Auflösung gewählt werden. Für die hier beschriebene Anwendung sind übliche Werte für $\beta$ = 5° - 15°, für NA = 0,15 - 0,45.

An diese Faseroptik kann sich wiederum eine Detektoranordnung 28, wie zuvor beschrieben, anschließen, oder, wenn anstelle der Detektoranordnung ein lichtempfindlicher Film zur Anwendung kommt, der Film, wie in FIG 2 aufgezeigt, an der Faseroptik vorbeibewegt werden.

**Ansprüche**

1. Zahnärztliche Röntgendiagnostikeinrichtung enthaltend
   a) einen Röntgenstrahler (3),
   b) Mittel zur Konversion der Röntgenstrahlung in sichtbare Strahlung,
   c) Mittel zur Bilddarstellung der sichtbaren Strahlung;
   d) als Mittel zur Konversion der Röntgenstrahlung ist eine gitterartig strukturierte Szintillatorplatte (10) vorgesehen, die eine Vielzahl flächiger und sich in Strahlungsrichtung erstreckender Szintillatorelemente (17) enthält, welche durch lichtdichte Wandungen (18) voneinander getrennt sind und deren Strahlungseintrittsflächen (L/B) im Verhältnis zur Fläche der sie begrenzenden Wandungen (W) sehr groß sind;
   e) die Szintillatorplatte weist in Strahlungsrichtung eine Höhe (H) auf, die mindestens das Doppelte der Abmessung (Breite, Länge oder Durchmesser) eines Szintillatorelementes (17) beträgt.

2. Zahnärztliche Röntgendiagnostikeinrichtung enthaltend
   a) einen Röntgenstrahler,
   b) Mittel zur Konversion der Röntgenstrahlung in sichtbare Strahlung,
   c) Mittel zur Bilddarstellung der sichtbaren Strahlung;
   d) als Mittel zur Konversion der Röntgenstrahlung ist eine unstrukturierte Szintillatorplatte (25) aus transparentem Szintillatormaterial vorgesehen, an die sich eine gitterartig strukturierte Faseroptik (26) mit relativ kleiner numerischer Apertur anschließt;
   e) die Faseroptik besteht aus einer Vielzahl von flächigen und sich räumlich in Strahlungsrichtung erstreckenden Einzelelementen (27).

3. Röntgendiagnostikeinrichtung nach Anspruch 1, **dadurch gekennzeichnet,** daß die Szintillatorplatte (10) aus lichtdichtem Material besteht, in der mit Szintillatormaterial gefüllte Kanäle (20), die im gefüllten Zustand die Szintillatorelemente (17) bilden, eingearbeitet sind.

4. Röntgendiagnostikeinrichtung nach Anspruch 3, **dadurch gekennzeichnet,** daß die Innenflächen (21) der Kanäle (20) vor dem Einbringen des Szintillatormaterials mit einer lichtreflektierenden Oberfläche versehen, vorzugsweise verspiegelt sind.

5. Röntgendiagnostikeinrichtung nach Anspruch 1 oder 2, **dadurch gekennzeichnet,** daß die einzelnen Elemente (17) rechteckigen Querschnitt haben.

6. Röntgendiagnstikeinrichtung nach Anspruch 5, **dadurch gekennzeichnet,** daß die einzelnen Elemente (17) gegeneinander versetzt angeordnet sind.

7. Röntgendiagnostikeinrichtung nach Anspruch 5, **dadurch gekennzeichnet,** daß die Elemente (17) im Querschnitt quadratisch sind und eine Kantenlänge von 50 $\mu$m aufweisen, und daß die Breite (W) der Wandungen der optischen Isolierung 5 $\mu$m und die Höhe (H) der Szintillatorplatte 500 $\mu$m beträgt.

8. Röntgendiagnostikeinrichtung nach einem der Ansprüche 1 bis 7, **dadurch gekennzeichnet,** daß zur Bilddarstellung eine Detektoranordnung mit Halbleiter-Elementen vorgesehen ist, wobei die Anzahl der einzelnen Szintillatorelemente (17) der Anzahl der vorhandenen Halbleiter-Elemente entspricht oder ein Mehrfaches dieser beträgt.

FIG 1

FIG 2

FIG 3

FIG 4

FIG 5

FIG 6

FIG 7

FIG 8

FIG 9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | DE-A-2 527 750 (SYBRON CORP.) * Seite 6, Zeile 6 - Seite 8, Zeile 22; Figuren 1-3,5 * | 1,2 | A 61 B 6/14 |
| A | | 5,7,8 | |
| Y | PATENT ABSTRACTS OF JAPAN Band 12, Nr. 319 (P-751)(3166), 30. August 1988; & JP - A - 63 85 484 (TOSHIBA CORP.) 15.04.1988 * ganze Zusammenfassung; Figur * | 1,2 | |
| A | idem | 3 | |
| A | US-A-4 304 998 (R.H. CUSHMAN) * Spalte 4, Zeile 16 - Spalte 6, Zeile 40; Figur 4 * | 1,2 | |

RECHERCHIERTE
SACHGEBIETE (Int. Cl.5)

A 61 B 6/00
G 03 B 42/00
G 01 T 1/00

Der vorliegende Recherchenbericht wurde für alle Patentansprüche erstellt

| Recherchenort | Abschlußdatum der Recherche | Prüfer |
|---|---|---|
| BERLIN | 02-04-1990 | WEIHS J.A. |